(19) **Europäisches Patentamt / European Patent Office / Office européen des brevets**

(11) **EP 3 936 035 A1**

(12) **EUROPEAN PATENT APPLICATION**
published in accordance with Art. 153(4) EPC

(43) Date of publication:
**12.01.2022 Bulletin 2022/02**

(21) Application number: **20766297.4**

(22) Date of filing: **10.02.2020**

(51) Int Cl.:
**A61B 5/02** (2006.01)  **A61B 5/1455** (2006.01)

(86) International application number:
**PCT/JP2020/005146**

(87) International publication number:
**WO 2020/179375 (10.09.2020 Gazette 2020/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.03.2019   JP 2019040897**

(71) Applicant: **Asahi Kasei Kabushiki Kaisha Tokyo 1000006 (JP)**

(72) Inventors:
• **TSUBOI Yuka**
  **Tokyo 100-0006 (JP)**
• **YAMADA Masashi**
  **Tokyo 100-0006 (JP)**

(74) Representative: **Hoffmann Eitle**
  **Patent- und Rechtsanwälte PartmbB**
  **Arabellastraße 30**
  **81925 München (DE)**

(54) **BIOLOGICAL INFORMATION MEASURING DEVICE**

(57)   The arterial blood oxygen saturation is estimated with high accuracy, irrespective of the measurement environment or the measurement position of a living body. A biological information measuring device includes: a first light emitter configured to irradiate a biotissue in which blood flows with light of a first wavelength; a second light emitter arranged in proximity to the first light emitter and configured to irradiate the biotissue with light of a second wavelength different from the first wavelength; a first photodetector configured to photodetect the light of the first wavelength and the light of the second wavelength transmitted through or reflected from the biotissue; a second photodetector arranged in proximity to the first photodetector and configured to photodetect the light of the first and second wavelengths; and a processor configured to calculate a change over time in the extinction coefficient of the light of the first wavelength based on the photodetection amount of the light of the first wavelength photodetected with each of the first and second photodetectors, calculate a change over time in the extinction coefficient of the light of the second wavelength based on the photodetection amount of the light of the second wavelength photodetected with each of the first and second photodetectors, and calculate the arterial blood oxygen saturation from the changes over time in the extinction coefficients of the light of the first and second wavelengths.

FIG. 1

**Description**

Technical Field

The present disclosure relates to a biological information measuring device.

Background Art

As one of the biomarkers indicating the degree of oxygen contained in blood of a living body, the percutaneous arterial blood oxygen saturation ($SpO_2$, sometimes referred to as "arterial blood oxygen saturation below") is known. The arterial blood oxygen saturation can be generally estimated by irradiating a biotissue with red light and infrared light, and then measuring transmitted light transmitted through the capillaries. The light of two wavelengths has a difference in the extinction characteristics to oxidized hemoglobin ($O_2Hb$) and reduced hemoglobin (HHb). The arterial blood oxygen saturation can be estimated by utilizing this difference in the extinction characteristics. Specifically, a probe (sensor) is attached mainly to a finger or the like, light of two different wavelengths (red light and infrared light) is emitted to the finger or the like, and then the intensity of transmitted light or reflected light of each light is measured. The saturation amount of oxidized hemoglobin is calculated based on the intensity of the transmitted light or the reflected light of the light of two different wavelengths.

For example, PTLS 1, 2 below disclose oximeters photodetecting red light and infrared light transmitted through a living body with a single photodetector to estimate the arterial blood oxygen saturation. The arterial blood oxygen saturation is usually determined by a ratio (theoretical value) between the concentration of oxidized hemoglobin ($O_2Hb$) and the total concentrations of oxidized hemoglobin ($O_2Hb$) and reduced hemoglobin (HHb) . However, the actual arterial blood oxygen saturation deviates from the above-described theoretical value. Thus, the devices disclosed in PTLS 1, 2 estimate the arterial blood oxygen saturation using a calibration equation calibrating the above-described theoretical value or a table in which the theoretical value and the arterial blood oxygen saturation are associated with each other, considering the scattering of the light in the biotissue.

Citation List

Patent Literatures

PTL 1: JP S62-102736 A
PTL 2: JP 2010-233908 A

Summary

However, the arterial blood oxygen saturation estimated using the calibration equation or the table described above does not sufficiently consider the scattering of the light in the living body, and thus still greatly deviates from the actual arterial blood oxygen saturation in some cases. In particular, when the arterial blood oxygen saturation is low, the deviation between the estimated arterial blood oxygen saturation and the actual arterial blood oxygen saturation tends to be larger.

Thus, it is an object of the present disclosure to provide a biological information measuring device capable of estimating the arterial blood oxygen saturation with high accuracy, irrespective of the measurement environment or the measurement position of a living body.

In order to solve the above-described problem, a biological information measuring device according to one aspect of the present disclosure includes: a first light emitter configured to irradiate a biotissue in which blood flows with light of a first wavelength; a second light emitter arranged in proximity to the first light emitter and configured to irradiate the biotissue with light of a second wavelength different from the first wavelength; a first photodetector configured to photodetect the light of the first wavelength and the light of the second wavelength transmitted through or reflected from the biotissue; a second photodetector arranged in proximity to the first photodetector and configured to photodetect the light of the first wavelength and the light of the second wavelength transmitted through or reflected from the biotissue; and a processor configured to calculate a change over time in the extinction coefficient of the light of the first wavelength based on the photodetection amount of the light of the first wavelength photodetected with each of the first photodetector and the second photodetector, calculate a change over time in the extinction coefficient of the light of the second wavelength based on the photodetection amount of the light of the second wavelength photodetected with each of the first photodetector and the second photodetector, and calculate the arterial blood oxygen saturation from the change over time in the extinction coefficient of the light of the first wavelength and the change over time in the extinction coefficient of the light of the second wavelength.

A biological information measuring device according to another aspect of the present disclosure includes: a first light emitter configured to irradiate a biotissue in which blood flows with light of a first wavelength; a second light emitter arranged in proximity to the first light emitter and configured to irradiate the biotissue with light of a second wavelength different from the first wavelength; a third light emitter configured to irradiate the biotissue with light of the first wavelength; a fourth light emitter arranged in proximity to the third light emitter and configured to irradiate the biotissue with light of the second wavelength; a photodetector configured to photodetect the light of the first wavelength and the light of the second wavelength transmitted through or reflected from the biotissue; and a processor configured to calculate a change over time in the extinction coefficient of the light of the first wavelength based on the photodetection amounts of the light of the first wavelength from the first light emitter and the light of the first wavelength from the third light emitter photodetected with the photodetector, calculate a change over time in the extinction coefficient of the light of the second wavelength based on the photodetection amounts of the light of the second wavelength from the second light emitter and the light of the second wavelength from the fourth light emitter photodetected with the photodetector, and calculate the arterial blood oxygen saturation from the change over time in the extinction coefficient of the light of the first wavelength and the change over time in the extinction coefficient of the light of the second wavelength.

The present disclosure makes it possible to estimate the arterial blood oxygen saturation with high accuracy, irrespective of the measurement environment or the measurement position of a living body.

Brief Description of Drawings

FIG. 1 is a block diagram illustrating a configuration example of a biological information measuring device according to a first embodiment of the present disclosure;

FIG. 2 is a plan view illustrating the arrangement of light emitters and photodetectors on the contact surface with a living body of the biological information measuring device according to the first embodiment of the present disclosure;

FIG. 3 is a schematic cross-sectional view illustrating a state where the light emitters and the photodetectors are arranged on a living body and the optical paths of light emitted from the light emitters in the biological information measuring device according to the first embodiment of the present disclosure;

FIG. 4 is a view illustrating amplitude fluctuations of the photodetection amount (intensity) of transmitted light over time of the photodetection amount (intensity $I_0$) of incident light entering a living body;

FIG. 5 is a plan view illustrating the arrangement of light emitters and a photodetector on the contact surface with a living body of a biological information measuring device according to a second embodiment of the present disclosure;

FIG. 6 is a plan view illustrating the arrangement of light emitters and photodetectors on the contact surface with a living body of a biological information measuring device according to a third embodiment of the present disclosure;

FIG. 7 is a schematic cross-sectional view illustrating a state where the light emitters and the photodetectors are arranged on a living body and the optical paths of light emitted from the light emitters in the biological information measuring device according to the third embodiment of the present disclosure;

FIG. 8A and 8B are plan views illustrating the arrangement of light emitters and photodetectors on the contact surface with a living body of a biological information measuring device according to a fourth embodiment of the present disclosure;

FIG. 9 is a plan view illustrating the arrangement of light emitters and a photodetector on the contact surface with a living body of a biological information measuring device according to a fifth embodiment of the present disclosure;

FIG. 10A and 10B are plan views illustrating the arrangement of light emitters and a photodetector on the contact surface with a living body of a biological information measuring device according to a sixth embodiment of the present disclosure; and

FIG. 11 is a plan view illustrating the arrangement of light emitters and a photodetector on the contact surface with a living body of a biological information measuring device according to a seventh embodiment of the present disclosure.

Description of Embodiments

Embodiments of the present disclosure will now be described with reference to the drawings. However, the embodiments described below are merely examples and are not intended to exclude the application of various modifications or technologies not clearly described below. The present disclosure can be implemented in various modifications (e.g., by combining each embodiment) without departing from the gist of the present disclosure. In the following description of the drawings, the same or similar parts are designated by the same or similar reference numerals. The drawings are schematic and do not necessarily match an actual dimension, ratio, or the like. In some cases, the dimensional relationships and ratios may be different between the drawings.

## 1. Conventional method for measuring arterial blood oxygen saturation (SpO$_2$)

First, a conventional method for measuring the arterial blood oxygen saturation (SpO$_2$) is described.

Conventionally, the arterial blood oxygen saturation (SpO$_2$) has been estimated using light of two wavelengths based on a difference in the absorption coefficients for the optical wavelengths between oxidized hemoglobin and reduced hemoglobin. The arterial blood oxygen saturation (SpO$_2$) estimated using the light of two wavelengths is represented by Equation (1) below.

[Equation 1]

$$SpO_2 = \frac{\varepsilon_{HHb,Red} - \varepsilon_{HHb,IR}R}{\varepsilon_{HHb,Red} - \varepsilon_{O_2Hb,Red} + [\varepsilon_{O_2Hb,IR} - \varepsilon_{HHb,IR}]R} \quad \cdots (1)$$

In which, in Expression (1), R is represented by Expression (2) below.

[Equation 2]

$$R = \frac{A_{Red}}{A_{IR}} = \frac{\log(I_{L,Red}/I_{H,Red})}{\log(I_{L,IR}/I_{H,IR})} \quad \cdots (2)$$

In Equation (2), $A_{Red}$ is the absorbance of red light Red, $A_{IR}$ is the absorbance of infrared light IR, $I_{L,Red}$ is the minimum amplitude of amplitude fluctuations over time of the photodetection amount (intensity) of the red light Red, $I_{H,Red}$ is the maximum amplitude of the amplitude fluctuations over time of the photodetection amount (intensity) of the red light Red, $I_{L,IR}$ is the minimum amplitude of amplitude fluctuations over time of the photodetection amount (intensity) of the infrared light IR, and $I_{H,IR}$ is the maximum amplitude of the amplitude fluctuations over time of the photodetection amount (intensity) of the infrared light IR. Herein, the minimum amplitude and the maximum amplitude of the amplitude fluctuations over time of the photodetection amount (intensity) of the red light Red mean the amplitude of the pulsation. In Equation (2), $\varepsilon_{O2Hb,Red}$ is the molar extinction coefficient of the red light Red to the oxidized hemoglobin (O$_2$Hb), $\varepsilon_{HHb,Red}$ is the molar extinction coefficient of the red light Red to the reduced hemoglobin (HHb), $\varepsilon_{O2Hb, IR}$ is the molar extinction coefficient of the infrared light to the oxidized hemoglobin (O$_2$Hb), and $\varepsilon_{HHb, IR}$ is the molar extinction coefficient of the infrared light IR to the reduced hemoglobin (HHb). In Equation (2), [O$_2$Hb] is the oxidized hemoglobin concentration and [HHb] is the reduced hemoglobin concentration.

Equation (1) is obtained from the Beer-Lambert law represented by Equation (3) below and the theoretical equation for the arterial oxygen saturation (SpO$_2$) represented by Equation (4).

[Equation 3]

$$A = -\log(\frac{I}{I_0}) = \varepsilon \cdot C \cdot d \quad \cdots (3)$$

In which, in Equation (3), A is the absorbance, $I_0$ is the intensity of incident light, I is the intensity of transmitted light, $\varepsilon$ is the molar extinction coefficient, C is the absorber molar concentration, and d is the distance (thickness of the absorber).

[Equation 4]

$$SpO_2 = \frac{[O_2Hb]}{[O_2Hb] + [HHb]} \quad \cdots (4)$$

In which, in Equation (4), [O$_2$Hb] is the oxidized hemoglobin concentration and [HHb] is the reduced hemoglobin concentration.

However, the Beer-Lambert law represented by Equation (3) is not a model considering the scattering of the light in a biological tissue (in particular, a biological tissue other than arterial blood, which is a pulsation component) through which the light is transmitted. Therefore, the arterial blood oxygen saturation estimated using Equation (1) (theoretical value estimated from Equation (1)) has a large deviation from the actual arterial blood oxygen saturation. Conventional SpO$_2$ measuring devices are so-called "transmissive" measuring devices and mostly measure the SpO$_2$ by irradiating a finger with measuring light. Since the finger has a large number of capillaries and an optical path difference in a living body due to a wavelength difference is small in the case of the transmissive measuring device, the SpO$_2$ was able to be measured with high accuracy by the use of the transmissive measuring device.

However, the measurement with the finger has posed a problem that peripheral blood vessels contract in a low temperature environment, resulting in a decrease in measurement accuracy. Further, there has been a problem that, when a sensor is attached to a position other than the finger, for example, to measure the SpO$_2$, the measurement accuracy further decreases particularly when a reflective measuring device is used, because the optical path difference in a living body for each wavelength increases.

Thus, the present disclosures have proposed a biological information measuring device and a biological information measuring method capable of measuring the SpO$_2$ with high accuracy at various positions of a living body.

Hereinafter, each embodiment describes in detail the biological information measuring device and the biological information measuring method.

2. First Embodiment

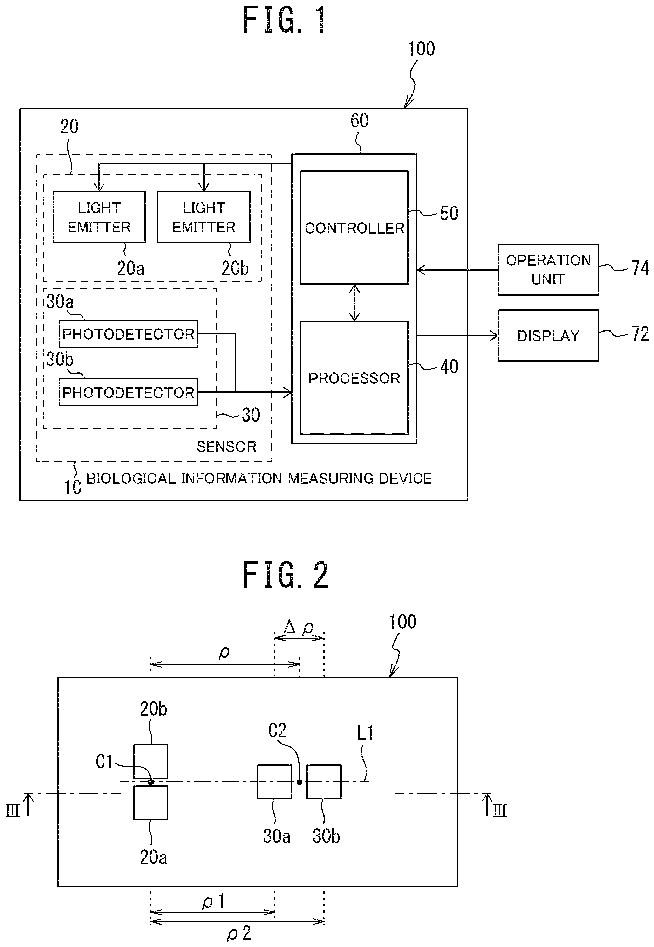

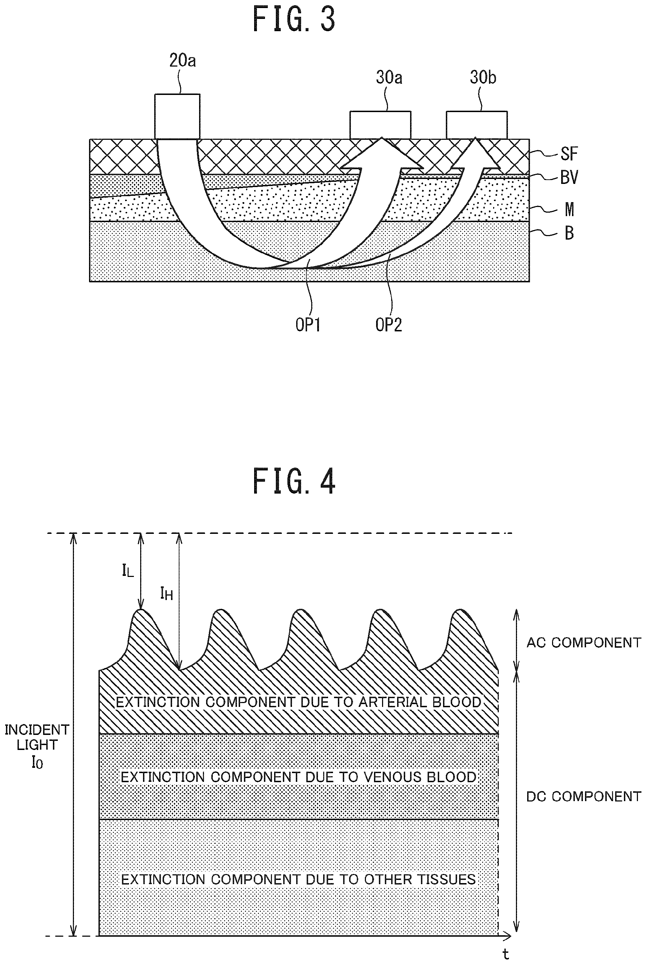

Hereinafter, a biological information measuring device and a biological information measuring method according to a first embodiment are described using FIG. 1 to FIG. 4. FIG. 1 is a block diagram illustrating the configuration of a biological information measuring device 100 according to the first embodiment. FIG. 2 is a plan view illustrating the arrangement of a light emitter 20 (light emitters 20a, 20b) and a photodetector 30 (photodetectors 30a, 30b) on the contact surface with a living body of the biological information measuring device 100 according to this embodiment. FIG. 3 illustrates a III-III cross section of FIG. 2 and is a cross-sectional schematic view illustrating a state where the light emitter 20 (20a, 20b) and the photodetector 30 (30a, 30b) are arranged on a living body and optical paths OP1, OP2 of light emitted from the light emitter 20. FIG. 4 is a view illustrating amplitude fluctuations of the photodetection amount (intensity) of transmitted light over time of the photodetection amount (intensity $I_0$) of incident light entering the living body.

[Biological information measuring device]

As illustrated in FIG. 1, the biological information measuring device 100 according to the first embodiment includes a sensor 10 having the light emitter 20 and the photodetector 30 and a microprocessor 60 having a processor 40 and a controller 50, for example. The biological information measuring device 100 is a pulse oximeter. The biological information measuring device 100 is configured so that the sensor 10 and the microprocessor 60 are housed in a housing, which is not illustrated, and the light emitter 20 and the photodetector 30 are exposed to the outside to be able to closely contact a living body, for example. The biological information measuring device 100 may also be connected to a display 72 displaying the arterial blood oxygen saturation (SpO$_2$) measured with the biological information measuring device 100 or an operation unit 74 operating the biological information measuring device 100, for example. For the display 72, existing displays, such as a Liquid Crystal Display (LCD) and an Organic Electro-Luminescence Display (OLED), are usable, for example. For the operation unit 74, existing input devices, such as keyboards, touch panels, buttons, and voice input units, are usable. The biological information measuring device 100 may also include the display 72 and the operation unit 74, for example, inside.

The biological information measuring device 100 includes the light emitter 20a and the light emitter 20b as the light emitter 20 and the photodetector 30a and the photodetector 30b as the photodetector 30. In the biological information measuring device 100, light is emitted from the light emitters 20a, 20b and light of different wavelengths transmitted through a living body is photodetected with each of the photodetectors 30a, 30b. The light emitters 20a, 20b and the photodetectors 30a, 30b are individually arranged so that the distance between the photodetector 30a and the light emitters 20a, 20b is different from the distance between the photodetector 30b and the light emitters 20a, 20b.

More specifically, the biological information measuring device 100 photodetects the light of different wavelengths at a plurality of places (e.g., two places of the arrangement positions of the photodetectors 30a, 30b) through a living body. The biological information measuring device 100 measures the attenuation of the light quantity at each wavelength of the oxidized hemoglobin (O$_2$Hb) and the reduced hemoglobin (HHb) in the living body between the arrangement positions of the photodetectors 30a, 30b from the photodetection amounts measured with the photodetectors 30a, 30b. The biological information measuring device 100 measures the arterial blood oxygen saturation (SpO$_2$) with higher accuracy utilizing the slope of the absorbance described above.

Hereinafter, each unit of the biological information measuring device 100 is described in detail.

(Light emitter)

As illustrated in FIG. 1 to FIG. 3, the light emitter 20 includes the light emitter 20a and the light emitter 20b. The light emitter 20 may also include three or more light emitters.

The light emitter 20a is a first light emitter emitting light of a first wavelength (e.g., red light). The light emitter 20a is arranged in close contact with a living body in which blood flows and irradiates the living body with the light of the first wavelength.

The light emitter 20b is a second light emitter emitting light of a second wavelength (e.g., infrared light) having a wavelength different from the first wavelength. The light emitter 20b is arranged in close contact with a living body in which blood flows and irradiates the living body with the light of the second wavelength as with the light emitter 20a. The light emitter 20b is arranged in proximity to the light emitter 20a.

The light emitters 20a, 20b alternately emit the light in a predetermined cycle.

The light emitters 20a, 20b contain Light Emitting Diodes (LEDs), small lasers, and the like, for example, as light emitting elements.

The light emitted from the light emitters 20a, 20b is preferably red light or infrared light to be used for measuring the reduced hemoglobin. The wavelength (first wavelength) of the light emitted from the light emitter 20a and the wavelength (second wavelength) of the light emitted from the light emitter 20b are preferably 400 nm or more and 1000 nm or less and more preferably 650 nm or more and 950 nm or less.

The wavelength of the light emitted from the light emitters 20a, 20b of 400 nm or more is preferable because the absorption of the light into the hemoglobin is not excessively high and an effect on the living body decreases. The wavelength of the light emitted from the light emitters 20a, 20b of 650 nm or more is preferable because the photodetection amounts in the photodetectors 30a, 30b are sufficient.

The wavelength of the light emitted from the light emitters 20a, 20b of 1000 nm or less is preferable because the absorption of the light into water in a biotissue is not excessively high and the photodetection amounts in the photodetectors 30a, 30b are sufficient. The wavelength of the light emitted from the light emitters 20a, 20b of 950 nm or less is preferable because an effect on the absorption of the light into the water in the biotissue further decreases and noise caused by the absorption of the light decreases.

The biological information measuring device 100 measures the arterial blood oxygen saturation ($SpO_2$) utilizing a difference in the light absorption coefficient between the oxidized hemoglobin ($O_2Hb$) and the reduced hemoglobin (HHb). Therefore, it is preferable that the light emitted from the light emitter 20a is red light and the light emitted from the light emitter 20b is infrared light.

(Photodetector)

As illustrated in FIG. 1 to FIG. 3, the photodetector 30 includes the photodetector 30a and the photodetector 30b. The photodetector 30 may also include three or more photodetectors.

The photodetector 30a photodetects the light of the first wavelength (e.g., red light) and the light of the second wavelength (e.g., infrared light) transmitted through or reflected from a biotissue.

The photodetector 30b is arranged in proximity to the photodetector 30a and photodetects the light of the first wavelength (e.g., red light) and the light of the second wavelength (e.g., infrared light) transmitted through or reflected from the biotissue.

The photodetectors 30a, 30b photodetect both the red light and the infrared light alternately emitted from the light emitters 20a, 20b while dividing photodetection time. When the photodetectors 30a, 30b include each of an infrared light photodetection element and a red light photodetection element, the above-described division of the photodetection time is not performed.

The photodetectors 30a, 30b include a photodiode (PD), for example, as the photodetection element.

As illustrated in FIG. 2, the photodetectors 30a, 30b are arranged such that a distance ($\rho1$) between the light emitters 20a, 20b and the photodetector 30a is different from a distance ($\rho2$) between the light emitters 20a, 20b and the photodetector 30b. Hereinafter, $\rho1$ is defined as a first photodetector distance and $\rho2$ is defined as a second photodetector distance. The distance $\rho1$ between the photodetector 30a and the light emitter 20a and the light emitter 20b and the distance $\rho2$ between the photodetector 30b and the light emitter 20a and the light emitter 20b each are preferably 1 mm or more and 100 mm or less and more preferably 1 mm or more and 50 mm or less. When the distances $\rho1$, $\rho2$ are 1 mm or more, the assumption of the photon diffusion equation in a living body that the distances are sufficiently larger than the mean free step is satisfied, and thus the measurement can be performed with high accuracy. Further, the distances $\rho1$, $\rho2$ of 100 mm or less are preferable because the photodetection amount of diffused light is sufficient. Further, the distances $\rho1$, $\rho2$ of 40 mm or less are preferable because the sensor 10 is reduced in size, making it easy to attach the sensor 10 to the surface of a living body.

As illustrated in FIG. 2, the photodetector 30b is arranged on a straight line L1 passing through a midpoint C1 between the light emitter 20a and the light emitter 20b and the photodetector 30a. Thus, the photodetectors 30a, 30b and the midpoint C1 between the light emitter 20a and the light emitter 20b are arranged on the same straight line, for example. The photodetector 30b is arranged in the same direction as that of the photodetector 30a with respect to the midpoint C1. In this case, as illustrated in FIG. 3, for example, an optical path OP1, through which light is transmitted from the light emitter 20a to the photodetector 30a through a living body, and an optical path OP2, through which light is transmitted from the light emitter 20a to the photodetector 30b through the living body, are close to each other. More specifically, the biotissue compositions are closer to each other in the optical path OP1 (path in the living body) of the red light between the light emitter 20a and the photodetector 30a and the optical path OP2 (path in the living body) of the red light between the light emitter 20a and the photodetector 30b. Similarly, the biotissue compositions are closer to each other in an optical path (path in the living body) of the infrared light between the light emitter 20b and the photodetector 30a and an optical path (path in the living body) of the infrared light between the light emitter 20b and the photodetector 30b. Therefore, an error of the photodetection amount due to an optical path difference decreases, and thus the arterial blood oxygen saturation ($SpO_2$) can be estimated with higher accuracy.

(Microprocessor)

The microprocessor 60 has the processor 40, the controller 50, an analog-to-digital converter (A/D converter), which is not illustrated, and a digital-to-analog converter (D/A converter), which is not illustrated, for example. The analogue-to-digital converter (A/D converter) converts an analogue signal input to the microprocessor 60, for example, into a digital signal. The digital-to-analogue converter (D/A converter) converts a digital signal output from the microprocessor 60 into an analogue signal.

(Controller)

The controller 50 has, for example, a drive circuit (not illustrated) driving the light emitters 20a, 20b, a VI converter (not illustrated) converting a voltage output from the photodetectors 30a, 30b into a current, and an amplifier circuit (not illustrated) amplifying an output of the VI converter. The controller 50 further has a drive controller (not illustrated) controlling the drive circuit and a processing controller (not illustrated) controlling the processor 40. The controller 50 controls the drive controller and the processing controller based on an operation instruction input from the outside through the operation unit 74.

(Processor)

The processor 40 performs arithmetic processing of the arterial blood oxygen saturation ($SpO_2$) when outputs corresponding to the photodetection amounts from the photodetectors 30a, 30b are input thereinto. Further, the processor 40 outputs the arterial blood oxygen saturation ($SpO_2$) as a result of the arithmetic processing to the external display 72.

The processor 40 calculates a change over time in the extinction coefficient of the light of the first wavelength based on the photodetection amount of the light of the first wavelength photodetected with each of the photodetectors 30a, 30b, and calculates a change over time in the extinction coefficient of the light of the second wavelength based on the photodetection amount of the light of the second wavelength photodetected with each of the photodetectors 30a, 30b. The processor 40 calculates the arterial blood oxygen saturation from the change over time in the extinction coefficient of the light of the first wavelength of and the change over time in the extinction coefficient of the light of the second wavelength. Herein, the change over time in the extinction coefficient of the light of the first wavelength and the change over time in the extinction coefficient of the light of the second wavelength indicate changes over time in the extinction coefficients of the light with respect to the distance $\rho 1$ between the light emitters 20a, 20b and the photodetector 30a and the distance p2 between the light emitters 20a, 20b and the photodetector 30b.

Specifically, the processor 40 calculates a ratio $R_{\mu a}$ of the extinction coefficients due to pulsation components of the light of the first wavelength and the light of the second wavelength as the change over time in the extinction coefficient of the light of the first wavelength and the change over time in the extinction coefficient of the light of the second wavelength, and then calculates the arterial blood oxygen saturation ($SpO_2$) based on the extinction coefficient ratio $R_{\mu a}$. The extinction coefficient ratio $R_{\mu a}$ can be calculated using the amplitude fluctuations of the light of the first wavelength and the light of the second wavelength (maximum amplitude and minimum amplitude, details are described below). For example, when the light of the first wavelength is the red light Red and the light of the second wavelength is the infrared light IR, the processor 40 calculates a ratio $R_{\mu a}$ ($\mu_{a,Red}/\mu_{a,IR}$) between the absorption coefficient $\mu_{a,Red}$ of the red light Red and the absorption coefficient $\mu_{a,IR}$ of the infrared light IR from the changes over time in the extinction coefficients of the red light Red and the infrared light IR. In the absorption coefficient, a light scattering element is removed, and thus an $SpO_2$ value closer to the actual measurement system than that in a conventional principle can be obtained.

Hereinafter, the arithmetic processing in the processor 40 is described in detail.

As illustrated in FIG. 3, the light emitted from the light emitters 20a, 20b is emitted to the skin surface of a living body, and then transmitted light (emitted light) transmitted through the living body while being reflected and scattered is photodetected with the photodetectors 30a, 30b, respectively, in the sensor 10 of the biological information measuring device 100. As illustrated in FIG. 3, the light emitted from the light emitters 20a, 20b can be transmitted through layers, such as a subcutaneous fat layer SF, a blood vessel layer BV, a muscle layer M, and a bone B, in the living body. At this time, as illustrated in FIG. 4, the incident light is partly absorbed by the arteries, veins, and other body tissues present in the living body and is observed as emitted light in the light emitters 20a, 20b. As illustrated in FIG. 4, the amplitude of the photodetection amount (intensity) of the transmitted light vibrates between a maximum amplitude $I_H$ and a minimum amplitude $I_L$ of a pulsation component (Alternating Current (AC) component).

The processor 40 calculates the ratio $R_{\mu a}$ between the absorption coefficient $\mu_{a,Red}$ of the red light Red and the absorption coefficient $\mu_{a,IR}$ of the infrared light IR, and then calculates the arterial blood oxygen saturation (SpO$_2$) based on the absorption coefficient ratio $R_{\mu a}$ as described above.

The processor 40 acquires the following eight parameters (intensity of transmitted light) from the photodetectors 30a, 30b to determine the absorption coefficient ratio $R_{\mu a}$. $I_{H,Red,P1}$, $I_{L,Red,P1}$, $I_{H,IR,P1}$, $I_{L,IR,P1}$, $I_{H,Red,P2}$, $I_{L,Red,P2}$, $I_{H,IR,P2}$, $I_{L,IR,P2}$

$I_{H,Red,P1}$ and $I_{L,Red,P1}$ are the maximum amplitude ($I_{H,Red,P1}$) and the minimum amplitude ($I_{L,Red,P1}$), respectively, of the amplitude fluctuations over time of the photodetection amount (intensity) of the red light Red photodetected with the photodetector arranged at a position P1 where the distance from the light emitter of the red light Red is ρ1. $I_{H,Red,P1}$ and $I_{L,Red,P1}$ are measured by a combination of the light emitter 20a and the photodetector 30a arranged at the position P1 where the distance from the light emitter 20a is ρ1.

$I_{H,IR,P1}$ and $I_{L,IR,P1}$ are the maximum amplitude ($I_{H,IR,P1}$) and the minimum amplitude ($I_{L,IR,P1}$), respectively, of the amplitude fluctuations over time of the photodetection amount (intensity) of the infrared light IR photodetected with the photodetector arranged at the position P1 where the distance from the light emitter of the infrared light IR is ρ1. $I_{H,IR,P1}$ and $I_{L,IR,P1}$ are measured by a combination of the light emitter 20b and the photodetector 30a arranged at the position P1 where the distance from the light emitter 20b is ρ1.

$I_{H,Red,P2}$ and $I_{L,Red,P2}$ are the maximum amplitude ($I_{H,Red,P2}$) and the minimum amplitude ($I_{L,Red,P2}$) of the amplitude fluctuations over time of the photodetection amount (intensity) of the red light Red photodetected with the photodetector 30b arranged at a position P2 where the distance from the light emitter of the red light Red is p2. $I_{H,Red,P1}$ and $I_{L,Red,P1}$ are measured by a combination of the light emitter 20a and the photodetector 30b arranged at the position P2 where the distance from the light emitter 20a is p2.

$I_{H,IR,P2}$ and $I_{L,IR,P2}$ are the maximum amplitude ($I_{H,IR,P2}$) and the minimum amplitude ($I_{L,IR,P2}$) of the amplitude fluctuations over time of the photodetection amount (intensity) of the infrared light IR photodetected with the photodetector 30b arranged at the position P2 where the distance from the light emitter of the infrared light IR is p2. $I_{H,IR,P2}$ and $I_{L,IR,P2}$ $I_{L,IR,P2}$ are measured by a combination of the light emitter 20b and the photodetector 30b arranged at the position P2 where the distance from the light emitter 20b is p2.

Specifically, the biological information measuring device 100 according to this embodiment calculates the arterial blood oxygen saturation (SpO$_2$) from Equation (5) below. The SpO$_2$ (theoretical value) calculated based on Equation (5) considers an effect of the scattering of the light in the living body, and therefore a deviation from the actual SpO$_2$ value decreases.

[Equation 5]

$$SpO_2 = \frac{\varepsilon_{HHb,Red} - \varepsilon_{HHb,IR} R_{\mu_a}}{\varepsilon_{HHb,Red} - \varepsilon_{O_2Hb,Red} + \left[\varepsilon_{O_2Hb,IR} - \varepsilon_{HHb,IR}\right] R_{\mu_a}} \quad \cdots (5)$$

In which, in Equation (5), $\varepsilon_{HHb,Red}$ indicates the molar extinction coefficient of the red light Red to the reduced hemoglobin (HHb), $\varepsilon_{HHb,IR}$ indicates the molar extinction coefficient of the infrared light IR to the reduced hemoglobin (HHb), $\varepsilon_{O2Hb,Red}$ indicates the molar extinction coefficient of the red light Red to the oxidized hemoglobin (O$_2$Hb), and $\varepsilon_{O2Hb,IR}$ indicates the molar extinction coefficient of the infrared light IR to the oxidized hemoglobin (O$_2$Hb). $R_{\mu a}$ is a ratio ($\mu_{a,Red}$, $\mu_{a,IR}$) of the extinction coefficients due to the pulsation (AC) components of the red light Red and the infrared light IR and is represented by Equation (6) below.

[Equation 6]

$$R_{\mu a} = \frac{\mu_{a,Red}}{\mu_{a,IR}}$$

$$= \frac{1 - h \cdot \lambda_{IR}}{1 - h \cdot \lambda_{Red}} \left\{ \frac{\log\left((I_{L,Red,P1} \cdot I_{H,Red,P2})/(I_{H,Red,P1} \cdot I_{L,Red,P2})\right) - 2\Delta\rho/(\ln 10 \cdot \rho)}{\log\left((I_{L,IR,P1} \cdot I_{H,IR,P2})/(I_{H,IR,P1} \cdot I_{L,IR,P2})\right) - 2\Delta\rho/(\ln 10 \cdot \rho)} \right\}^2 \quad \ldots (6)$$

In Equation (6), $\mu_{a,Red}$ indicates the absorption coefficient of the red light Red and $\mu_{a,IR}$ indicates the absorption coefficient of the infrared light IR. h indicates the normalized gradient (from $6.3 \times 10^{-4}$ [mm$^{-1}$/nm], $\lambda_{IR}$ indicates the wavelength of the infrared light IR, $\lambda_{Red}$ indicates the wavelength of the red light Red, $\Delta\rho$ is a difference between the first photodetector distance $\rho1$ and the second photodetector distance p2, and p indicates the average between the first photodetector distance $\rho1$ and the second photodetector distance p2. $\Delta\rho$ is a difference (p2 - $\rho1$) between the distance $\rho1$ between the light emitter 20 (20a, 20b) and the photodetector 30a and the distance p2 between the light emitter 20 (20a, 20b) and the photodetector 30b and indicates the distance between the two measurement points P1, P2 (distance between the positions of the photodetectors 30a, 30b). $\rho$ indicates the distance between a midpoint C2 between the two measurement points P1, P2 (photodetectors 30a, 30b) and the light emitter 20.

As is understood from Equation (6), the absorption coefficient ratio $R_{\mu a}$ is calculated using the eight parameters obtained from the photodetectors 30, 30b.

Equation (5) is an equation in which an R value (ratio ($A_{Red}/A_{IR}$) between the absorbance $A_{IR}$ of the infrared light IR and the absorbance $A_{Red}$ of the red light Red) in a conventional theoretical equation for the calculation of the SpO$_2$ represented in Equation (1) is replaced by the extinction coefficient ratio $R_{\mu a}$.

The absorbances A ($A_{Red}$, $A_{IR}$) are parameters including the light scattering element. Unlike the absorption of light, the scattering of the light does not greatly reflect an effect of increase/decrease in hemoglobin. Therefore, the light scattering element should be originally an element to be removed in the SpO$_2$ calculation. In contrast thereto, the extinction coefficient ratio $R_{\mu a}$ is a parameter from which the light scattering element has been removed. Therefore, the SpO$_2$ calculated using the extinction coefficient ratio $R_{\mu a}$ obtained with the biological information measuring device 100 according to this embodiment is a value considering the effect of the scattering of the light in biotissues other than the arterial blood.

[Effects of First Embodiment]

The biological information measuring device 100 according to the first embodiment calculates the arterial blood oxygen saturation by calculating each of the changes over time in the absorption coefficients of the two kinds of light based on the photodetection amounts of the light of the two wavelengths (light of the first wavelength and light of the second wavelength) detected at the two measurement points . Therefore, the scattering of the light into body compositions other than the pulsation component (arterial blood) is considered, and thus the deviation of the calculated SpO$_2$ from the actual SpO$_2$ value decreases.

[Modification of First Embodiment]

In the biological information measuring device 100 according to the first embodiment, the case of detecting the light of the two wavelengths using the two measurement points (photodetectors) is described but the present disclosure is not limited thereto. More specifically, the biological information measuring device 100 according to the first embodiment may detect the light of the two wavelengths using three or more light emitters.

3. Second Embodiment

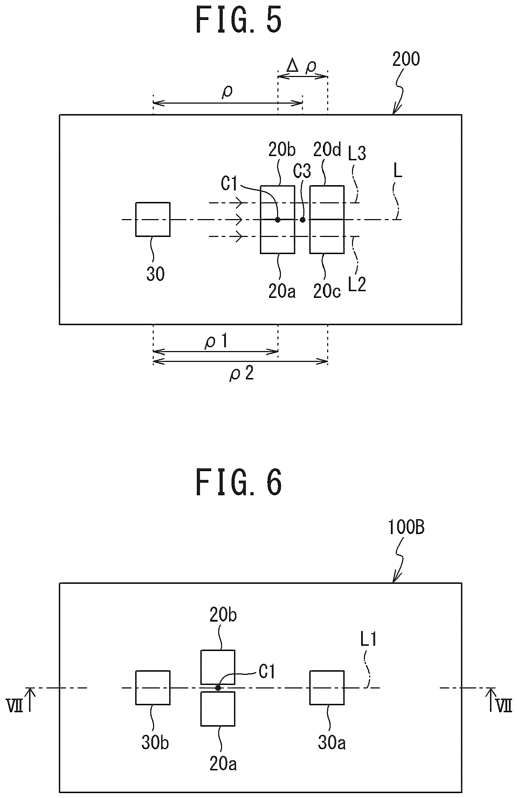

Hereinafter, a biological information measuring device 200 according to a second embodiment is described using FIG. 5, referring to FIG. 1 and FIG. 4. FIG. 5 is a plan view illustrating the arrangement of the light emitter 20 (light emitters 20a, 20b, 20c, 20d) and the photodetector 30 on the contact surface with a living body of the biological information measuring device 200 according to the second embodiment.

The biological information measuring device 200 is similar to the biological information measuring device 100, except that the arrangement of the light emitter 20 and the photodetector 30 is changed from that of the biological information measuring device 100 of the first embodiment, and thus the processing in the processor 40 is correspondingly partly changed, and therefore the description is omitted.

(Photodetector)

As illustrated in FIG. 5, the photodetector 30 contains a single photodetector.

The photodetector 30 photodetects the light of the first wavelength (e.g., red light) and the light of the second wavelength (e.g., infrared light) transmitted through or reflected from a biotissue. Details are described below.

The photodetector 30 photodetects each of the red light and the infrared light emitted in turn from the light emitters 20a, 20b, 20c, and 20d while dividing the photodetection time.

(Light emitter)

As illustrated in FIG. 5, the light emitter 20 includes the light emitter 20a, the light emitter 20b, a light emitter 20c, and a light emitter 20d. The light emitter 20a and the light emitter 20b are similar to the light emitter 20a and the light emitter 20b of the first embodiment, and therefore the description thereof is omitted.

The light emitter 20c is a third light emitter emitting the light of the first wavelength (e.g., red light) as with the light of the first wavelength emitted from the light emitter 20a. The light emitter 20c is arranged in close contact with a living body in which blood flows and irradiates the living body with the light of the first wavelength.

The light emitter 20d is a fourth light emitter emitting the light of the second wavelength (e.g., infrared light) as with the light of the second wavelength emitted from the light emitter 20b. The second wavelength is different from the first wavelength. More specifically, the light emitter 20c and the light emitter 20d emit light of wavelengths different from each other. The light emitter 20d is arranged in close contact with a living body in which blood flows as with the light emitter 20a to the light emitter 20c and irradiates the living body with the light of the second wavelength. The light emitter 20d is arranged in proximity to the light emitter 20c.

With respect to the light emitters 20a to 20d, the light emitters 20a, 20b are arranged in proximity to each other at the position P1 and the light emitters 20c, 20d are arranged in proximity to each other at the position P2. The light emitters 20a, 20b are arranged such that the distance ($\rho$1) between the light emitters 20a, 20b and the photodetector 30 is different from the distance (p2) between the light emitters 20c, 20d and the photodetector 30.

The light emitter 20 may include five or more light emitters. In this case, as with the light emitters 20a, 20b and the light emitters 20c, 20d described above, the five or more light emitters are arranged such that the light emitters emitting light of different wavelengths are arranged in proximity to each other.

As illustrated in FIG. 5, the light emitter 20c is arranged on a straight line L2 parallel to the straight line L passing through the photodetector 30 and the midpoint C1 between the light emitters 20a, 20b and passing through the light emitter 20a. The light emitter 20c is arranged in the same direction as that of the light emitter 20a with respect to the photodetector 30. The light emitter 20d is arranged on a straight line L3 parallel to the straight line L and passing through the light emitter 20b. The light emitter 20d is arranged in the same direction as that of the light emitter 20b with respect to the photodetector 30.

In this case, as with the first embodiment, the biotissue compositions on the optical paths are close to each other because the optical paths, through which light is transmitted from the light emitters 20a to 20d to the photodetector 30 through the living body, are close to each other. Therefore, the error of the photodetection amount due to the optical path difference decreases, and thus the arterial blood oxygen saturation (SpO$_2$) can be estimated with higher accuracy.

(Processor)

The processor 40 calculates a change over time in the extinction coefficient of the light of the first wavelength (e.g., red light) based on the photodetection amounts of the light of the first wavelength from the light emitter 20a, 20c photodetected with the photodetector 30. Further, the processor 40 calculates a change over time in the extinction coefficient of the light of the second wavelength (e.g., infrared light) based on the photodetection amounts of the light of the second wavelength from the light emitters 20b, 20d photodetected with the photodetector 30. The processor 40 calculates the arterial blood oxygen saturation from the change over time in the extinction coefficient of the light of the first wavelength and the change over time in the extinction coefficient of the light of the second wavelength.

The processor 40 calculates the ratio $R_{\mu a}$ between the absorption coefficient $\mu_{a,Red}$ of the red light Red and the absorption coefficient $\mu_{a,IR}$ of the infrared light IR from the change over time in the extinction coefficient of the light of the first wavelength and the change over time in the extinction coefficient of the light of the second wavelength, and then calculates the arterial blood oxygen saturation (SpO$_2$) based on the extinction coefficient ratio $R_{\mu a}$. The processor 40 acquires the following eight parameters (intensity of transmitted light) from the photodetector 30 to determine the absorption coefficient ratio $R_{\mu a}$. $I_{H,Red,P1}$, $I_{L,Red,P1}$, $I_{H,IR,P1}$, $I_{L,IR,P1}$, $I_{H,Red,P2}$, $I_{L,Red,P2}$, $I_{H,IR,P2}$, $I_{L,IR,P2}$

$I_{H,Red,P1}$ and $I_{L,Red,P1}$ are measured by a combination of the light emitter 20a emitting the red light Red and the photodetector 30 arranged at the position P1 where the distance from the light emitter 20a is $\rho$1.

$I_{H,IR,P1}$ and $I_{L,IR,P1}$ are measured by a combination of the light emitter 20b emitting the infrared light IR and the photodetector 30 arranged at the position P1 where the distance from the light emitter 20b is $\rho$1.

$I_{H,Red,P2}$ and $I_{L,Red,P2}$ are measured by a combination of the light emitter 20c emitting the red light Red and the photodetector 30 arranged at the position P2 where the distance from the light emitter 20c is p2.

$I_{H,IR,P2}$ and $I_{L,IR,P2}$ are measured by a combination of the light emitter 20d emitting the infrared light IR and the photodetector 30 arranged at the position P2 where the distance from the light emitter 20d is p2.

The processor 40 calculates the arterial blood oxygen saturation ($SpO_2$) from Equation (5) below as with the first embodiment.

[Equation 7]

$$SpO_2 = \frac{\varepsilon_{HHb,Red} - \varepsilon_{HHb,IR} R_{\mu_a}}{\varepsilon_{HHb,Red} - \varepsilon_{O_2Hb,Red} + [\varepsilon_{O_2Hb,IR} - \varepsilon_{HHb,IR}] R_{\mu_a}} \quad \cdots (5)$$

In which, in Equation (5), $\varepsilon_{HHb,Red}$ indicates the molar extinction coefficient of the red light Red to the reduced hemoglobin (HHb), $\varepsilon_{HHb,IR}$ indicates the molar extinction coefficient of the infrared light IR to the reduced hemoglobin (HHb), $\varepsilon_{O2Hb,Rea}$ indicates the molar extinction coefficient of the red light Red to the oxidized hemoglobin ($O_2$Hb), and $\varepsilon_{O2Hb,IR}$ indicates the molar extinction coefficient of the infrared light IR to the oxidized hemoglobin ($O_2$Hb) . $R_{\mu_a}$ is the ratio ($\mu_{a,Red}$, $\mu_{a,IR}$) of the extinction coefficients due to the AC components of the red light Red and the infrared light IR.

The biological information measuring device 100 according to this embodiment calculates the ratio $R_{\mu_a}$ ($\mu_{a,Red}/\mu_{a,IR}$) of the absorption coefficients of the red light and the infrared light based on the photodetection amounts (intensity) of the light of the first wavelength and the light of the second wavelength (light emitted from the light emitters 20a to 20d) photodetected with the photodetector 30 . In the second embodiment, the absorption coefficient ratio $R_{\mu_a}$ is calculated based on Equation (6) below as with the first embodiment.

[Equation 8]

$$R_{\mu a} = \frac{\mu_{a,Red}}{\mu_{a,IR}}$$
$$= \frac{1 - h \cdot \lambda_{IR}}{1 - h \cdot \lambda_{Red}} \left\{ \frac{\log((I_{L,Red,P1} \cdot I_{H,Red,P2})/(I_{H,Red,P1} \cdot I_{L,Red,P2})) - 2\Delta\rho/(\ln 10 \cdot \rho)}{\log((I_{L,IR,P1} \cdot I_{H,IR,P2})/(I_{H,IR,P1} \cdot I_{L,IR,P2})) - 2\Delta\rho/(\ln 10 \cdot \rho)} \right\}^2 \quad \cdots (6)$$

In Equation (6), $\mu_{a,Red}$ indicates the absorption coefficient of the red light Red and $\mu_{a,IR}$ indicates the absorption coefficient of the infrared light IR. h indicates the normalized gradient (from $6.3 \times 10^{-4}$ [mm$^{-1}$/nm], $\lambda_{IR}$ indicates the wavelength of the infrared light IR, $\lambda_{Red}$ indicates the wavelength of the red light Red, $\Delta\rho$ is a difference between the first photodetector distance $\rho$1 and the second photodetector distance p2, and p indicates the average between the first photodetector distance $\rho$1 and the second photodetector distance $\rho$2. $\Delta\rho$ is a difference (p2 - $\rho$1) between the distance $\rho$1 between the light emitter 20 (20a, 20b) and the photodetector 30 and the distance p2 between the light emitter 20 (20c, 20d) and the photodetector 30 and indicates the distance between two light emitting points (distance between the light emitters 20a, 20b and the light emitters 20c, 20d). $\rho$ indicates the distance between a midpoint C3 between the two light emitting points (light emitters 20a, 20b and light emitters 20c, 20d) and the photodetector 30.

[Effects of Second Embodiment]

The biological information measuring device 200 according to the second embodiment considers the scattering of the light into body compositions other than the pulsation component (arterial blood) as with the biological information measuring device 100 according to the first embodiment, and thus the deviation from the actual $SpO_2$ value decreases.

4. Third Embodiment

The first embodiment gives the example of the biological information measuring device 100 in which the photodetector 30b is arranged on the straight line L1 passing through the midpoint C1 between the light emitter 20a and the light emitter 20b and the photodetector 30a and in the same direction as that of the photodetector 30a with respect to the midpoint C1, but the present disclosure is not limited to this configuration.

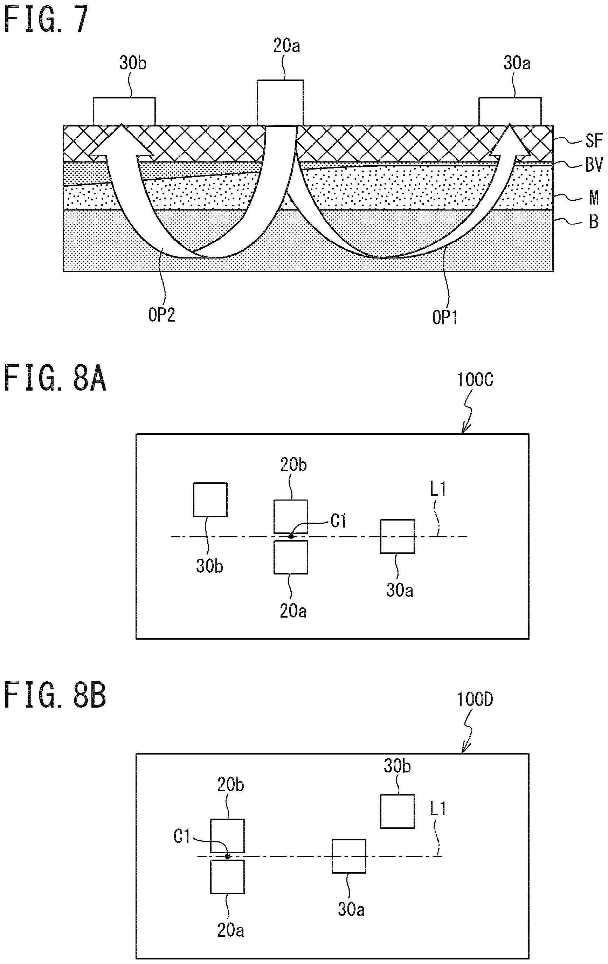

For example, in a biological information measuring device 100B according to a third embodiment, the photodetector 30b may be arranged on the straight line L1 passing through the midpoint C1 between the light emitter 20a and the light emitter 20b and the photodetector 30a and in the opposite direction to the photodetector 30a with respect to the midpoint C1 as illustrated in FIG. 6 and FIG. 7. FIG. 7 illustrates a VII-VII cross section of FIG. 6 and is a cross-sectional schematic view illustrating a state where the light emitter 20 (20a, 20b) and the photodetector 30 (30a, 30b) are arranged on a living body and the optical paths OP1, OP2 of the light emitted from the light emitter 20. As illustrated in FIG. 7, the light emitted from the light emitters 20a, 20b can be transmitted through the layers, such as the subcutaneous fat layer SF, the blood vessel layer BV, the muscle layer M, and the bone B, in the living body.

Such a biological information measuring device 100B has a high possibility that the biotissue compositions on the optical paths (optical paths from the light emitters 20a, 20b to the photodetectors 30a, 30b), through which light is transmitted through the living body, are different from each other. Therefore, there is a possibility that the error due to the optical path difference is larger than that in the biological information measuring device 100 of the first embodiment. However, the measurement principle considering the effect of the scattering of the light in a living body described in the first embodiment is also applicable to the biological information measuring device 100B.

5. Fourth Embodiment

The first embodiment and the third embodiment give the examples of the biological information measuring devices 100, 100B, respectively, in which the photodetector 30b is arranged on the straight line L1 passing through the midpoint C1 between the light emitter 20a and the light emitter 20b and the photodetector 30a, but the present disclosure is not limited to this configuration.

Biological information measuring devices 100C, 100D according to a fourth embodiment are configured as illustrated in a configuration example of FIG. 8A or FIG. 8B, respectively, so that the photodetector 30b is arranged at a position other than the positions on the straight line L1 passing through the midpoint C1 between the light emitter 20a and the light emitter 20b and the photodetector 30a.

In the biological information measuring device 100C illustrated in FIG. 8A, the photodetector 30a and the photodetector 30b are arranged on the opposite sides to each other with respect to the midpoint C1 in plan view (in FIG. 8A, separately arranged in a right side region and a left side region with respect to the midpoint C1) .

On the other hand, as illustrated in FIG. 8B, in the biological information measuring device 100D, the photodetector 30a and the photodetector 30b are arranged on the same side (in FIG. 8B, right side region with respect to the midpoint C1) with respect to the midpoint C1 in plan view. In the biological information measuring device 100D, the photodetector 30a and the photodetector 30b may be arranged in the left side region with respect to the midpoint C1 in FIG. 8B.

Also in this case, there is a possibility that the error due to the optical path difference is larger than that in the biological information measuring device 100 of the first embodiment as with the biological information measuring device 100B according to the third embodiment. However, the measurement principle considering the effect of the scattering of the light in the living body described in the first embodiment is also applicable to the biological information measuring devices 100C, 100D.

6. Fifth Embodiment

The second embodiment describes the biological information measuring device 200 in which the light emitter 20c is arranged on the straight line L2 parallel to the straight line L passing through the photodetector 30 and the midpoint C1 between the light emitters 20a, 20b and passing through the light emitter 20a and arranged in the same direction as that of the light emitter 20a with respect to the photodetector 30, but the present disclosure is not limited to this configuration.

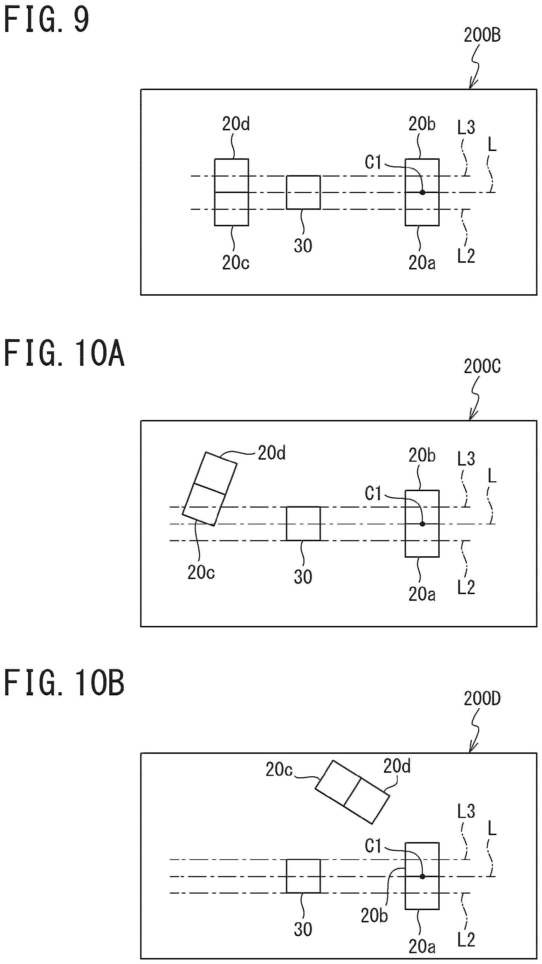

For example, in a biological information measuring device 200B according to a fifth embodiment, the light emitter 20c is arranged on the straight line L2 parallel to the straight line L passing through the photodetector 30 and the midpoint C1 between the light emitters 20a, 20b and passing through the light emitter 20a and arranged on the opposite side to the light emitter 20a with respect to the photodetector 30 as illustrated in FIG. 9. In the biological information measuring device 200B, the light emitter 20d is arranged on the straight line L2 parallel to the straight line L and passing through the light emitter 20b and arranged on the opposite side to the light emitter 20b with respect to the photodetector 30 as illustrated in FIG. 9.

Such a biological information measuring device 200B has a possibility that the error due to the optical path difference is larger than that in the biological information measuring device 200 of the second embodiment. However, the measurement principle considering the effect of the scattering of the light in the living body described in the second embodiment is also applicable to the biological information measuring device 200B.

7. Sixth Embodiment

The second embodiment and the fifth embodiment give the examples of the biological information measuring devices 200, 200B, respectively, in which the light emitter 20c is arranged on the straight line L2 parallel to the straight line L passing through the photodetector 30 and the midpoint C1 and passing through the light emitter 20a and the light emitter 20d is arranged on the straight line L3 parallel to the straight line L and passing through the light emitter 20b, but the present disclosure is not limited to this configuration.

Biological information measuring devices 200C, 200D according to a sixth embodiment are configured as illustrated in a configuration example of FIG. 10A or FIG. 10B, respectively, so that the light emitters 20c, 20d are individually arranged at positions other than positions on the straight lines L2, L3.

In the biological information measuring device 200C illustrated in FIG. 10A, the light emitters 20a, 20b and the light emitters 20c, 20d are arranged on the opposite sides to each other with respect to the photodetector 30 in plan view (in FIG. 10A, separately arranged in a right side region and a left side region with respect to the photodetector 30).

On the other hand, as illustrated in FIG. 10B, in the biological information measuring device 200D, the light emitters 20a, 20b and the light emitters 20c, 20d are arranged on the same side with respect to the photodetector 30 (in FIG. 10B, right side region with respect to the photodetector 30). In the biological information measuring device 200D, the light emitters 20a, 20b and the light emitters 20c, 20d may be arranged in the left side region with respect to the photodetector 30 in FIG. 10B.

Also in this case, there is a possibility that the error due to the optical path difference is larger than that in the biological information measuring device 200 of the second embodiment as with the biological information measuring device 200B according to the fifth embodiment. However, the measurement principle considering the effect of scattering of the light in a living body described in the second embodiment is also applicable to the biological information measuring devices 200C, 200D.

8. Seventh Embodiment

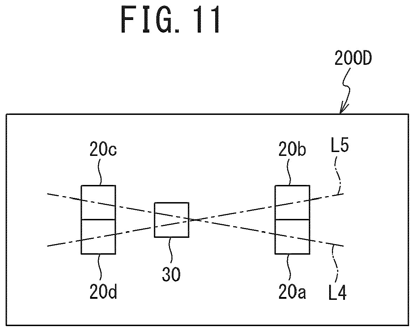

In a biological information measuring device 200D according to a seventh embodiment, the light emitter 20c may be arranged on a straight line L4 passing through the photodetector 30 and the light emitter 20a and may be arranged on the opposite side to the light emitter 20a with respect to the photodetector 30 as illustrated in FIG. 11. The light emitter 20d may be arranged on a straight line L5 passing through the photodetector 30 and the light emitter 20b and may be arranged on the opposite side to the light emitter 20b with respect to the photodetector 30.

In such a biological information measuring device 200D, there is a possibility that the error due to the optical path difference is larger than that in the biological information measuring device 200 of the second embodiment. However, the measurement principle considering the effect of scattering of the light in the living body described in the second embodiment is also applicable to the biological information measuring device 200D.

The specific configurations of the present disclosure are described above with reference to the embodiments but the scope of the present disclosure is not limited to the illustrative embodiments illustrated in the drawings and described above and also includes all embodiments that provide advantageous effects equivalent to those intended by the present disclosure. Further, the scope of the present disclosure is not limited to combinations of the features of the present invention defined by Claims but should be defined by any desired combination of specific features among all the disclosed features.

Reference Signs List

C1, C2, C3 midpoint
L, L1, L2, L3, L4, L5 straight line
10 sensor
20, 20a, 20b, 20c, 20d light emitter
30, 30a, 30b photodetector
40 processor
50 controller
60 microprocessor
72 display
74 operation unit
100, 100B, 100C, 200, 200B to 200D biological information measuring device

**Claims**

1. A biological information measuring device comprising:

   a first light emitter configured to irradiate a biotissue in which blood flows with light of first wavelength;
   a second light emitter arranged in proximity to the first light emitter and configured to irradiate the biotissue with light of a second wavelength different from the first wavelength;

a first photodetector configured to photodetect the light of the first wavelength and the light of the second wavelength transmitted through or reflected from the biotissue;

a second photodetector arranged in proximity to the first photodetector and configured to photodetect the light of the first wavelength and the light of the second wavelength transmitted through or reflected from the biotissue; and

a processor configured to calculate a change over time in an extinction coefficient of the light of the first wavelength based on a photodetection amount of the light of the first wavelength photodetected with each of the first photodetector and the second photodetector, calculate a change over time in an extinction coefficient of the light of the second wavelength based on a photodetection amount of the light of the second wavelength photodetected with each of the first photodetector and the second photodetector, and calculate an arterial blood oxygen saturation from the change over time in the extinction coefficient of the light of the first wavelength and the change over time in the extinction coefficient of the light of the second wavelength.

2. The biological information measuring device according to claim 1, wherein
the first photodetector and the second photodetector, and the first light emitter and the second light emitter are individually arranged such that a distance $\rho 1$ between the first and the second light emitters and the first photodetector is different from a distance $\rho 2$ between the first and the second light emitters and the second photodetector.

3. The biological information measuring device according to claim 2, wherein
the second photodetector is arranged on a straight line L1 passing through the first photodetector and a midpoint between the first light emitter and the second light emitter.

4. The biological information measuring device according to claim 3, wherein
the second photodetector is arranged in a same direction as a direction of the first photodetector with respect to the midpoint.

5. The biological information measuring device according to any one of claims 1 to 4, wherein
a distance between the first light emitter and the first and the second photodetectors and a distance between the second light emitter and the first and the second photodetectors are 1 mm or more and 100 mm or less.

6. The biological information measuring device according to any one of claims 1 to 5, wherein
the first wavelength and the second wavelength are 400 nm or more and 1000 nm or less.

7. The biological information measuring device according to claim 6, wherein
the processor is configured to calculate a ratio $R_{\mu a}$ of the extinction coefficients due to pulsation components of the light of the first wavelength and the light of the second wavelength based on the change over time in the extinction coefficient of the light of the first wavelength and the change over time in the extinction coefficient of the light of the second wavelength, and calculate the arterial blood oxygen saturation based on the extinction coefficient ratio $R_{\mu a}$.

8. The biological information measuring device according to claim 7, wherein

the first light emitter is configured to emit red light as the light of the first wavelength,
the second light emitter is configured to emit infrared light as the light of the second wavelength, and
the processor is configured to calculate the ratio $R_{\mu a}$ of the extinction coefficients due to the pulsation components of the red light and the infrared light from Expression (6) below,
[Equation 1]

$$\mathbf{R}_{\mu a} = \frac{\mu_{a,\text{Red}}}{\mu_{a,\text{IR}}}$$
$$= \frac{1-h\cdot\lambda_{\text{IR}}}{1-h\cdot\lambda_{\text{Red}}}\left\{\frac{\log((I_{\text{L,Red,P1}}\cdot I_{\text{H,Red,P2}})/(I_{\text{H,Red,P1}}\cdot I_{\text{L,Red,P2}}))-2\Delta\rho/(\ln10\cdot\rho)}{\log((I_{\text{L,IR,P1}}\cdot I_{\text{H,IR,P2}})/(I_{\text{H,IR,P1}}\cdot I_{\text{L,IR,P2}}))-2\Delta\rho/(\ln10\cdot\rho)}\right\}^2 \quad \ldots (6)$$

wherein, in Equation (6), $\mu_{a,\text{Red}}$ indicates an absorption coefficient of the red light Red and $\mu_{a,\text{IR}}$ indicates an absorption coefficient of the infrared light IR and h is a normalized gradient, $\lambda_{\text{IR}}$ is a wavelength of the infrared light IR, $\lambda_{\text{Red}}$ is a wavelength of the red light Red, $\Delta\rho$ is a difference between the distance between the first and the second light emitters and the first photodetector and the distance between the first and the second light emitters and the second photodetector, $\rho$ is an average between the distance between the first and the second light emitters and the first photodetector and the distance between the first and the second light emitters and the second photodetector, $I_{H,Red,P1}$ is a maximum amplitude of amplitude fluctuations over time of photodetection intensity of the red light photodetected with the first photodetector, $I_{L,Red,P1}$ is a minimum amplitude of the amplitude fluctuations over time of the photodetection intensity of the red light photodetected with the first photodetector, $I_{H,IR,P1}$ is a maximum amplitude of amplitude fluctuations over time of photodetection intensity of the infrared light photodetected with the first photodetector, $I_{L,IR,P1}$ is a minimum amplitude of the amplitude fluctuations over time of the photodetection intensity of the infrared light photodetected with the first photodetector, $I_{H,Red,P2}$ is a maximum amplitude of amplitude fluctuations over time of photodetection intensity of the red light photodetected with the second photodetector, $I_{L,Red,P2}$ is a minimum amplitude of the amplitude fluctuations over time of the photodetection intensity of the red light photodetected with the second photodetector, $I_{H,IR,P2}$ is a maximum amplitude of amplitude fluctuations over time of photodetection intensity of the infrared light photodetected with the second photodetector, and $I_{L,IR,P2}$ is a minimum amplitude of the amplitude fluctuations over time of the photodetection intensity of the infrared light photodetected with the second photodetector.

9. The biological information measuring device according to any one of claims 6 to 8, wherein

the processor is configured to calculate the arterial blood oxygen saturation from Equation (5) below,
[Equation 2]

$$SpO_2 = \frac{\varepsilon_{HHb,Red} - \varepsilon_{HHb,IR}R_{\mu_a}}{\varepsilon_{HHb,Red} - \varepsilon_{O_2Hb,Red} + \left[\varepsilon_{O_2Hb,IR} - \varepsilon_{HHb,IR}\right]R_{\mu_a}} \quad \cdots (5)$$

wherein, in Equation (5), $\varepsilon_{HHb,Red}$ is a molar extinction coefficient of the red light to reduced hemoglobin, $\varepsilon_{HHb,IR}$ is a molar extinction coefficient of the infrared light to the reduced hemoglobin, $\varepsilon_{O2Hb,Red}$ is a molar extinction coefficient of the red light to oxidized hemoglobin, $\varepsilon_{O2Hb,IR}$ is a molar extinction coefficient of the infrared light IR to the oxidized hemoglobin, and $R_{\mu a}$ is the ratio of the extinction coefficients due to the pulsation components of the red light and the infrared light.

10. The biological information measuring device according to claim 1 comprising:

three or more photodetectors including the first photodetector and the second photodetector, arranged in proximity to one another, and configured to photodetect the light of the first wavelength and the light of the second wavelength, wherein
the processor is configured to calculate a change over time in an extinction coefficient of the light of the first wavelength based on a photodetection amount of the light of the first wavelength photodetected with each of the three or more photodetectors, calculate a change over time in an extinction coefficient of the light of the second wavelength based on a photodetection amount of the light of the second wavelength photodetected with each of the three or more photodetectors, and calculate the arterial blood oxygen saturation from the change over time in the extinction coefficient of the light of the first wavelength and the change over time in the extinction coefficient of the light of the second wavelength.

11. A biological information measuring device comprising:

a first light emitter configured to irradiate a biotissue in which blood flows with light of a first wavelength;
a second light emitter arranged in proximity to the first light emitter and configured to irradiate the biotissue with light of a second wavelength different from the first wavelength;
a third light emitter configured to irradiate the biotissue with light of the first wavelength;
a fourth light emitter arranged in proximity to the third light emitter and configured to irradiate the biotissue with light of the second wavelength;
a photodetector configured to photodetect the light of the first wavelength and the light of the second wavelength transmitted through or reflected from the biotissue; and
a processor configured to calculate a change over time in an extinction coefficient of the light of the first wavelength based on photodetection amounts of the light of the first wavelength from the first light emitter and the light of the first wavelength from the third light emitter photodetected with the photodetector, calculate a change over time in an extinction coefficient of the light of the second wavelength based on photodetection amounts of the light of the second wavelength from the second light emitter and the light of the second wavelength from the fourth light emitter photodetected with the photodetector, and calculate an arterial blood oxygen saturation from the change over time in the extinction coefficient of the light of the first wavelength and the change over time in the extinction coefficient of the light of the second wavelength.

12. The biological information measuring device according to claim 11, wherein
the photodetector, and the first light emitter, the second light emitter, the third light emitter, and the fourth light emitter are arranged such that a distance $\rho 1$ between the first and the second light emitters and the photodetector is different from a distance p2 between the third and the fourth light emitters and the photodetector.

13. The biological information measuring device according to claim 12, wherein

the third light emitter is arranged on a second straight line L2 parallel to a straight line L passing through the photodetector and a midpoint between the first light emitter and the second light emitter and passing through the first light emitter, and
the fourth light emitter is arranged on a third straight line L3 parallel to the straight line L passing through the photodetector and the midpoint between the first light emitter and the second light emitter and passing through the second light emitter.

14. The biological information measuring device according to claim 12, wherein

the third light emitter is arranged on a straight line L1 passing through the photodetector and the first light emitter, and
the fourth light emitter is arranged on a straight line L2 passing through the photodetector and the second light emitter.

15. The biological information measuring device according to claim 13 or 14, wherein

the third light emitter is arranged in a same direction as a direction of the first light emitter with respect to the photodetector, and
the fourth light emitter is arranged in a same direction as a direction of the second light emitter with respect to the photodetector.

16. The biological information measuring device according to any one of claims 11 to 15, wherein
the first wavelength and the second wavelength are 400 nm or more and 1000 nm or less.

17. The biological information measuring device according to claim 16, wherein
the processor is configured to calculate a ratio $R_{\mu a}$ of the extinction coefficients due to pulsation components of the light of the first wavelength and the light of the second wavelength based on the change over time in the extinction coefficient of the light of the first wavelength and the change over time in the extinction coefficient of the light of the second wavelength, and calculate the arterial blood oxygen saturation based on the extinction coefficient ratio $R_{\mu a}$.

18. The biological information measuring device according to claim 17, wherein

the first light emitter and the third light emitter are configured to emit red light,
the second light emitter and the fourth light emitter are configured to emit infrared light, and
the processor is configured to calculate the ratio $R_{\mu a}$ of the extinction coefficients due to the pulsation components of the red light and the infrared light from Expression (6) below,
[Equation 3]

$$R_{\mu a} = \frac{\mu_{a,\text{Red}}}{\mu_{a,\text{IR}}}$$
$$= \frac{1-h \cdot \lambda_{\text{IR}}}{1-h \cdot \lambda_{\text{Red}}} \left\{ \frac{\log((I_{L,\text{Red},P1} \cdot I_{H,\text{Red},P2})/(I_{H,\text{Red},P1} \cdot I_{L,\text{Red},P2})) - 2\Delta\rho/(\ln 10 \cdot \rho)}{\log((I_{L,\text{IR},P1} \cdot I_{H,\text{IR},P2})/(I_{H,\text{IR},P1} \cdot I_{L,\text{IR},P2})) - 2\Delta\rho/(\ln 10 \cdot \rho)} \right\}^2 \quad ...(6)$$

wherein, in Equation (6), $\mu_{a,\text{Red}}$ indicates an absorption coefficient of the red light Red and $\mu_{a,\text{IR}}$ indicates the absorption coefficient of the infrared light IR and h is a normalized gradient, $\lambda_{\text{IR}}$ is a wavelength of the infrared

light IR, $\lambda_{Red}$ is a wavelength of the red light Red, $\Delta\rho$ is a difference between a distance between the first light emitter and the second light emitter and the photodetector and a distance between the third light emitter and the fourth light emitter and the photodetector, $\rho$ is an average between the distance between the first light emitter and the second light emitter and the photodetector and the distance between the third light emitter and the fourth light emitter and the photodetector, $I_{H,Red,P1}$ is a maximum amplitude of amplitude fluctuations over time of photodetection intensity of the red light from the first light emitter photodetected with the photodetector, $I_{L,Red,P1}$ is a minimum amplitude of the amplitude fluctuations over time of the photodetection intensity of the red light from the first light emitter photodetected with the photodetector, $I_{H,IR,P1}$ is a maximum amplitude of amplitude fluctuations over time of photodetection intensity of the infrared light from the second light emitter photodetected with the photodetector, $I_{L,IR,P1}$ is a minimum amplitude of the amplitude fluctuations over time of the photodetection intensity of the infrared light from the second light emitter photodetected with the photodetector, $I_{H,Red,P2}$ is a maximum amplitude of amplitude fluctuations over time of photodetection intensity of the red light from the third light emitter photodetected with the photodetector, $I_{L,Red,P2}$ is a minimum amplitude of the amplitude fluctuations over time of the photodetection intensity of the red light from the third light emitter photodetected with the photodetector, $I_{H,IR,P2}$ is a maximum amplitude of amplitude fluctuations over time of photodetection intensity of the infrared light from the fourth light emitter photodetected with the photodetector, and $I_{L,IR,P2}$ is a minimum amplitude of the amplitude fluctuations over time of the photodetection intensity of the infrared light from the fourth light emitter photodetected with the photodetector.

19. The biological information measuring device according to any one of claims 16 to 18, wherein

the processor is configured to calculate the arterial blood oxygen saturation from Equation (5) below,
[Equation 4]

$$SpO_2 = \frac{\varepsilon_{HHb,Red} - \varepsilon_{HHb,IR}R_{\mu_a}}{\varepsilon_{HHb,Red} - \varepsilon_{O_2Hb,Red} + [\varepsilon_{O_2Hb,IR} - \varepsilon_{HHb,IR}]R_{\mu_a}} \quad \cdots (5)$$

wherein, in Equation (5), $\varepsilon_{HHb,\,Red}$ is a molar extinction coefficient of the red light to reduced hemoglobin, $\varepsilon_{HHb,\,IR}$ is a molar extinction coefficient of the infrared light to the reduced hemoglobin, $\varepsilon_{O2Hb,\,Red}$ is a molar extinction coefficient of the red light to oxidized hemoglobin, $\varepsilon_{O2Hb,\,IR}$ is a molar extinction coefficient of the infrared light to the oxidized hemoglobin, and $R_{\mu_a}$ is the ratio of the extinction coefficients due to the pulsation components of the red light and the infrared light.

20. The biological information measuring device according to claim 11 comprising:

five or more light emitters including the first light emitter, the second light emitter, the third light emitter, and the fourth light emitter and configured to emit the light of the first wavelength or the light of the second wavelength, wherein
the processor is configured to calculate a change over time in the extinction coefficient of the light of the first wavelength based on the photodetection amount of the light of the first wavelength photodetected with the photodetector, calculate a change over time in the extinction coefficient of the light of the second wavelength based on the photodetection amount of the light of the second wavelength photodetected with the photodetector, and calculate the arterial blood oxygen saturation from the change over time in the extinction coefficient of the light of the first wavelength and the change over time in the extinction coefficient of the light of the second wavelength.

# FIG. 1

# FIG. 2

# FIG. 3

# FIG. 4

# FIG. 5

# FIG. 6

# FIG. 7

# FIG. 8A

# FIG. 8B

# FIG. 9

200B

20d          20b    L3    L
                     C1
20c          30     20a    L2

# FIG. 10A

200C

20d
              20b    L3    L
              C1
20c    30     20a    L2

# FIG. 10B

200D

20c    20d
                     L3    L
              C1
       30     20b
              20a    L2

# FIG. 11

## EP 3 936 035 A1

<table>
<tr><td colspan="2" align="center">INTERNATIONAL SEARCH REPORT</td><td colspan="2">International application No.<br>PCT/JP2020/005146</td></tr>
</table>

**A. CLASSIFICATION OF SUBJECT MATTER**
Int. Cl. A61B5/02(2006.01)i, A61B5/1455(2006.01)i
FI: A61B5/1455, A61B5/02 310B

According to International Patent Classification (IPC) or to both national classification and IPC

**B. FIELDS SEARCHED**

Minimum documentation searched (classification system followed by classification symbols)
Int. Cl. A61B5/02, A61B5/1455

Documentation searched other than minimum documentation to the extent that such documents are included in the fields searched
Published examined utility model applications of Japan        1922-1996
Published unexamined utility model applications of Japan    1971-2020
Registered utility model specifications of Japan            1996-2020
Published registered utility model applications of Japan    1994-2020

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y<br>A | JP 2006-75354 A (CHUNICHI DENSHI CO., LTD.) 23 March 2006, claims, paragraphs [0012], [0028]-[0030], fig. 2, 3 | 1-4, 10<br>5-7, 9, 17, 19<br>5 |
| Y | WO 98/23916 A1 (OMRON CORP.) 04 June 1998, claims, page 6, line 12 to line 17, page 12, line 12 to page 13, line 1, fig. 11B | 5-7, 9 |
| X<br>Y<br>A | JP 4-215742 A (HEWLETT-PACKARD CO.) 06 August 1992, claims, paragraphs [0071]-[0073], fig. 8-10 | 11-16, 20<br>6-7, 9, 17, 19<br>18 |
| A | JP 2015-519556 A (MAYO FOUNDATION FOR MEDICAL EDUCATION AND RESEARCH) 09 July 2015, claims, paragraphs [0094], [0095], all drawings | 1-20 |

☒ Further documents are listed in the continuation of Box C.   ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>19.03.2020 | Date of mailing of the international search report<br>31.03.2020 |
|---|---|
| Name and mailing address of the ISA/<br>    Japan Patent Office<br>    3-4-3, Kasumigaseki, Chiyoda-ku,<br>    Tokyo 100-8915, Japan | Authorized officer<br><br>Telephone No. |

Form PCT/ISA/210 (second sheet) (January 2015)

**INTERNATIONAL SEARCH REPORT**

International application No.

PCT/JP2020/005146

C (Continuation).    DOCUMENTS CONSIDERED TO BE RELEVANT

| Category* | Citation of document, with indication, where appropriate, of the relevant passages | Relevant to claim No. |
|---|---|---|
| A | US 2004/0116789 A1 (BOAS, David Alan et al.) 17 June 2004, claims, paragraphs [0053]-[0057], figures | 1-20 |

Form PCT/ISA/210 (continuation of second sheet) (January 2015)

<div align="center">

**INTERNATIONAL SEARCH REPORT**

Information on patent family members

</div>

| | International application No. |
|---|---|
| | PCT/JP2020/005146 |

| Patent Documents referred to in the Report | Publication Date | Patent Family | Publication Date |
|---|---|---|---|
| JP 2006-75354 A | 23.03.2006 | (Family: none) | |
| WO 98/23916 A1 | 04.06.1998 | EP 942260 A1 claims, paragraphs [0028], [0064]-[0068], fig. 11B JP 3035791 B2 | |
| JP 4-215742 A | 06.08.1992 | US 5285783 A claims, column 15, lines 43-67, fig. 8-10 US 5285784 A US 5188108 A EP 442011 A1 EP 613652 A2 EP 613653 A2 DE 69029152 T2 DE 69030513 T2 DK 613652 T3 DK 613653 T3 | |
| JP 2015-519556 A | 09.07.2015 | US 2015/0109617 A1 claims, paragraphs [0156]-[0160], figures US 2015/0119661 A1 US 2017/0322084 A1 US 2019/0033135 A1 EP 2844980 A1 EP 2844983 A2 WO 2013/165887 A1 WO 2013/165888 A2 JP 2015-521282 A JP 2018-146589 A | |
| US 2004/0116789 A1 | 17.06.2004 | WO 2001/054573 A1 EP 1251770 A1 AU 3459001 A CA 2397840 A1 | |

Form PCT/ISA/210 (patent family annex) (January 2015)

**EP 3 936 035 A1**